(19) Europäisches Patentamt
European Patent Office
Office européen des brevets

(11) Veröffentlichungsnummer: **0 467 099 A2**

(12) # EUROPÄISCHE PATENTANMELDUNG

(21) Anmeldenummer: **91110325.7**

(22) Anmeldetag: **22.06.91**

(51) Int. Cl.5: **C07D 239/34**, C07D 239/52, C07D 239/60, C07D 251/16, C07D 251/22, C07D 251/30, C07D 401/12, //(C07D401/12, 241:00,239:00)

(30) Priorität: **05.07.90 DE 4021441**

(43) Veröffentlichungstag der Anmeldung: **22.01.92 Patentblatt 92/04**

(84) Benannte Vertragsstaaten: **BE CH DE FR GB IT LI NL**

(71) Anmelder: **BAYER AG**

**W-5090 Leverkusen 1 Bayerwerk(DE)**

(72) Erfinder: **Heinemann, Ulrich, Dr.**
**Am Sonnenhang 1**
**W-5653 Leichlingen 2(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen(DE)**
Erfinder: **Schmidt, Robert Rudolf, Dr.**
**Im Waldwinkel 110**
**W-5060 Bergisch Gladbach 2(DE)**

(54) **Substituierte Acrylophenone.**

(57) Beschrieben werden neue substituierte Acrylophenone der Formel (I)

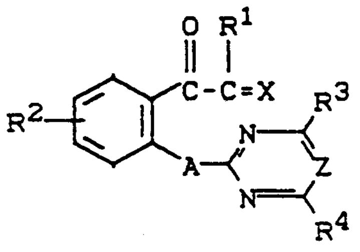

(I)

in denen
$R^1$, $R^2$, $R^3$, $R^4$, A, X und Z die in der Beschreibung angegebene Bedeutung haben, sowie mehrere Verfahren zu deren Herstellung. Verwendet werden die neuen Acrylophenone der Formel (I) als Herbizide.

EP 0 467 099 A2

Die Erfindung betrifft neue substituierte Acrylophenone, mehrere Verfahren zu ihrer Herstellung und ihre Verwendung als Herbizide.

Es ist bekannt, daß bestimmte substituierte Benzoesäurederivate, wie beispielsweise die Verbindung 2-[2-(4,6-Dimethoxy)-pyrimidinylthiol-benzoesäuremethylester herbizide Eigenschaften besitzen (vgl. z.B. EP 223406).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Acrylophenone der allgemeinen Formel (I),

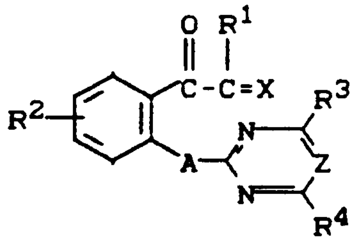

(I)

in welcher

| | |
|---|---|
| $R^1$ | für Wasserstoff oder Alkyl steht, |
| $R^2$ | für Wasserstoff, Halogen oder Alkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| A | für Sauerstoff oder Schwefel steht, |
| X | für einen Rest |

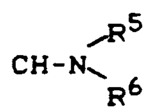

| | |
|---|---|
| | oder für einen Rest $P(Ar)_3$ steht und |
| Z | für Stickstoff oder für einen Rest $C-R^7$ steht, wobei entweder |
| $R^5$ | für Wasserstoff oder Alkyl steht und |
| $R^6$ | für Alkyl oder für gegebenenfalls substituiertes Phenyl steht oder |
| $R^5$ und $R^6$ | gemeinsam mit dem Stickstoffatom, an welches sie gebunden sind, für einen gegebenenfalls substituierten gesättigten Heterocyclus stehen, der gegebenenfalls weitere Heteroatome enthalten kann, |
| $R^7$ | für Wasserstoff oder Alkyl steht und |
| Ar | für gegebenenfalls substituiertes Phenyl steht, |

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Acrylophenone der allgemeinen Formel (I),

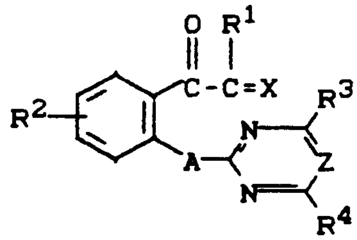

(I)

in welcher

| | |
|---|---|
| $R^1$ | für Wasserstoff oder Alkyl steht, |
| $R^2$ | für Wasserstoff, Halogen oder Alkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| A | für Sauerstoff oder Schwefel steht, |
| X | für einen Rest |

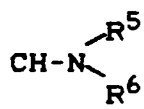

| | |
|---|---|
| | oder für einen Rest $P(Ar)_3$ steht und |
| Z | für Stickstoff oder für einen Rest C-$R^7$ steht, wobei entweder |
| $R^5$ | für Wasserstoff oder Alkyl steht und |
| $R^6$ | für Alkyl oder für gegebenenfalls substituiertes Phenyl steht oder |
| $R^5$ und $R^6$ | gemeinsam mit dem Stickstoffatom, an welches sie gebunden sind, für einen gegebenenfalls substituierten gesättigten Heterocyclus stehen, der gegebenenfalls weitere Heteroatome enthalten kann, |
| $R^7$ | für Wasserstoff oder Alkyl steht und |
| Ar | für gegebenenfalls substituiertes Phenyl steht, |

erhält, wenn man

(a) (Thio)Phenolderivate der Formel (II),

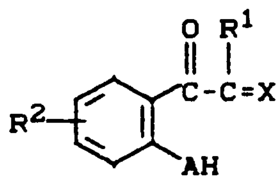

(II)

in welcher

$R^1$, $R^2$, A und X die oben angegebene Bedeutung haben,

mit Heterocyclen der Formel (III),

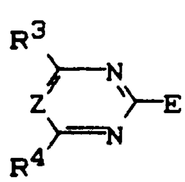

(III)

in welcher

E für eine elektronenanziehende Abgangsgruppe steht und
$R^3$, $R^4$ und Z die oben angegebene Bedeutung haben,

gegebenenfalls in Gegenwart eines Verdünnungsmittels und gegebenenfalls in Gegenwart eines Reaktionshilfsmittels umsetzt oder wenn man

(b) Ketone der Formel (IV),

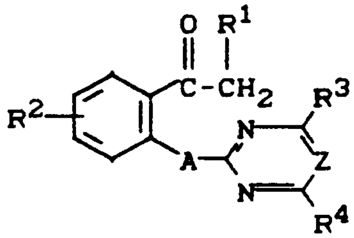

(IV)

in welcher

$R^1$, $R^2$, $R^3$, $R^4$, A und Z die oben angegebene Bedeutung haben,

mit Formamidacetalen der Formel (V),

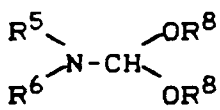

(V)

in welcher

$R^8$ für Alkyl steht und
$R^5$ und $R^6$ die oben angegebene Bedeutung haben, gegebenenfalls in Gegenwart eines Verdünnungsmittels umsetzt.

Schließlich wurde gefunden, daß die neuen substituierten Acrylophenone der Formel (I) gute herbizide, insbesondere auch selektiv herbizide Eigenschaften besitzen.

Überraschenderweise zeigen die erfindungsgemäßen substituierten Acrylophenone der allgemeinen Formel (I) neben einer deutlich höheren herbiziden Wirksamkeit gegenüber Unkräutern auch eine teilweise verbesserte Selektivität gegenüber wichtigen Kulturpflanzen im Vergleich zu den aus dem Stand der Technik bekannten substituierten Benzoesäurederivaten, wie beispielsweise die Verbindung 2-[2-(4,6-Dimethoxypyrimidinylthio)]-benzoesäuremethylester, welche chemisch und wirkungsmäßig naheliegende Verbindungen sind.

Die erfindungsgemäßen substituierten Acrylophenone sind durch die Formel (I) allgemein definiert.

Bevorzugte Substituenten bzw. Bereiche der in den oben und nachstehend erwähnten Formeln aufgeführten Reste werden im folgenden erläutert:

Alkyl steht einzeln oder in zusammengesetzten Resten für geradkettiges oder verzweigtes Alkyl mit 1 bis 4 und vorzugsweise mit 1 oder 2 Kohlenstoffatomen; beispielhaft und vorzugsweise seien genannt Methyl, Ethyl, n-und i-Propyl, n-, i-, s- und t-Butyl.

Alkoxy und Alkylthio stehen in den allgemeinen Formeln für geradkettiges oder verzweigtes Alkoxy bzw. Alkylthio mit 1 bis 4 und bevorzugt 1 oder 2 Kohlenstoffatomen; beispielhaft und vorzugsweise seien genannt: Methoxy, Ethoxy, n- und i-Propoxy, n-, i-, s- und t-Butoxy, Methylthio, Ethylthio, n- und i-Propylthio, n-, i-, s-und t-Butylthio.

Halogenalkyl, Halogenalkoxy und Halogenalkylthio stehen in den allgemeinen Formeln für geradkettiges oder verzweigtes Halogenalkyl, Halogenalkoxy bzw. Halogenalkylthio mit 1 bis 4 und bevorzugt mit 1 oder 2 Kohlenstoffatomen und jeweils 1 bis 9 und bevorzugt 1 bis 5, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Dichlormethyl, Difluorethyl, Trifluorethyl, Trichlorethyl, Trifluorchlorethyl, Chlorbutyl, Fluorbutyl und insbesondere Difluormethyl, Trifluormethyl, Trichlormethyl, Dichlorfluor-methyl und Chlordifluor-methyl sowie die diesen entsprechenden Halogenalkoxyreste bzw. Halogenalkylthioreste.

Ein Heterocyclus in den allgemeinen Formeln steht für einen gegebenenfalls Alkyl-substituierten 5- bis 7-gliedrigen Ring, der 1 bis 3, bevorzugt 1 oder 2 gleiche oder verschiedene Heteroatome enthält. Als

Heteroatome seien vorzugsweise Sauerstoff, Schwefel und Stickstoff genannt; beispielhaft und vorzugsweise seien genannt: Pyrrolidinyl, Piperidinyl, Azepinyl, Morpholinyl, Thiazanyl und Pyrazanyl.

Halogen steht im allgemeinen als Substituent oder in den Resten Halogenalkyl, Halogenalkoxy und Halogenalkylthio für Fluor, Chlor, Brom und Iod, insbesondere für Fluor und Chlor.

Als Substituenten für Phenyl seien vorzugsweise genannt:
Halogen; geradkettiges oder verzweigtes Alkyl mit 1 bis 4 Kohlenstoffatomen und besonders bevorzugt Methyl und Ethyl (die beispielhafte Aufzählung entspricht der weiter oben gegebenen); geradkettiges oder verzweigtes Alkoxy und Akylthio mit jeweils 1 bis 4 Kohlenstoffatomen und besonders bevorzugt mit 1 oder 2 Kohlenstoffatomen (beispielhaft seien genannt: Methoxy, Ethoxy, n-und i-Propoxy, n-, i-, s- und t-Butoxy, Methylthio, Ethylthio, n- und i-Propylthio); geradkettiges oder verzweigtes Halogenalkyl, Halogenalkoxy und Halogenalkylthio mit vorzugsweise je 1 bis 4 Kohlenstoffatomen, besonders bevorzugt mit jeweils 1 oder 2 Kohlenstoffatomen und jeweils 1 bis 9, vorzugsweise 1 bis 5, gleichen oder verschiedenen Halogenatomen wie unter Halogen definiert (beispielhaft seien genannt: Fluormethyl, Chlormethyl, Brommethyl, Fluorethyl, Chlorethyl, Brommethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy, Trifluorchlorethoxy und Trifluormethylthio); Nitro und Cyano.

Die hier aufgeführten Definitionen gelten in entsprechender Weise auch für die im folgenden aufgeführten bevorzugten Kombinationen von Resten,

Bevorzugt sind Verbindungen der Formel (I), bei welchen

$R^1$ für Wasserstoff oder für geradkettiges oder verzweigtes Alkyl mit 1 bis 4 Kohlenstoffatomen steht,

$R^2$ für Wasserstoff, Fluor, Chlor, Brom, Jod oder für geradkettiges oder verzweigtes Alkyl mit 1 bis 4 Kohlenstoffatomen steht,

$R^3$ und $R^4$ unabhängig voneinander jeweils für Wasserstoff, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen stehen,

A für Sauerstoff oder Schwefel steht,

X für einen Rest

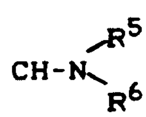

oder für einen Rest $P(Ar)_3$ steht und

Z für Stickstoff oder für einen Rest C-$R^7$ steht,
wobei entweder

$R^5$ für Wasserstoff oder für geradkettiges oder verzweigtes Alkyl mit 1 bis 4 Kohlenstoffatomen steht und

$R^6$ für geradkettiges oder verzweigtes Alkyl mit 1 bis 4 Kohlenstoffatomen oder für gegebenenfalls einfach oder mehrfach gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, oder

$R^5$ und $R^6$ gemeinsam mit dem Stickstoffatom, an welches sie gebunden sind, für einen gegebenenfalls einfach oder mehrfach, gleich oder verschieden durch geradkettiges oder verzweigtes Alkyl mit 1 bis 4 Kohlenstoffatomen substituierten gesättigten fünf- bis siebengliedrigen Heterocyclus stehen, der gegebenenfalls ein weiteres Heteroatom - insbesondere Stickstoff, Sauerstoff oder Schwefel - enthalten kann,

$R^7$ für Wasserstoff oder für geradkettiges oder verzweigtes Alkyl mit 1 bis 4 Kohlenstoffatomen steht und

Ar für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen.

Besonders bevorzugt sind Verbindungen der Formel (I), bei welchen

$R^1$ für Wasserstoff, Methyl oder Ethyl steht,

$R^2$ für Wasserstoff, Fluor, Chlor, Brom, Methyl oder Ethyl steht,

$R^3$ und $R^4$ unabhängig voneinander jeweils für Wasserstoff, Methyl, Ethyl, n- oder i-Propyl, Methoxy, Ethoxy, Methylthio, Ethylthio, Trifluormethyl, Difluormethyl, Trichlormethyl, Trifluormethoxy, Difluormethoxy, Trifluormethylthio, Trichlormethylthio, Chlordifluormethylthio oder Dichlorfluormethylthio stehen,

A für Sauerstoff oder Schwefel steht,

X für einen Rest

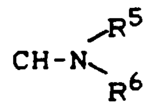

oder für einen Rest $P(Ar)_3$ steht und

Z für Stickstoff oder für einen Rest C-$R^7$ steht,

wobei entweder

$R^5$ für Methyl, Ethyl, sowie n- oder i-Propyl und

$R^6$ für Methyl, Ethyl, n- oder i-Propyl oder für gegebenenfalls einfach oder zweifach gleich oder verschieden substituiertes Phenyl steht, wobei als Substituenten infrage kommen: Fluor, Chlor, Brom, Methyl, Ethyl, Methoxy, Ethoxy oder Trifluormethyl oder

$R^5$ und $R^6$ gemeinsam mit dem Stickstoffatom, an welches sie gebunden sind, für einen gegebenenfalls ein- bis dreifach, gleich oder verschieden durch Methyl, Ethyl sowie n- oder i-Propyl substituierten Heterocyclus der Formel

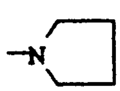 ; 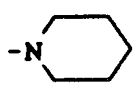 ; 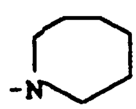 ; 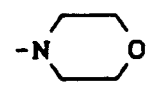 ;

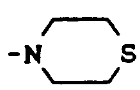 oder 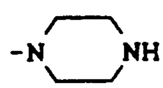

stehen,

$R^7$ für Wasserstoff, Methyl oder Ethyl steht und

Ar für gegebenenfalls einfach oder zweifach, gleich oder verschieden substituiertes Phenyl steht, wobei als Substituenten infrage kommen: Fluor, Chlor, Brom, Methyl, Ethyl, Methoxy, Ethoxy oder Trifluormethyl.

Im einzelnen seien außer den bei den Herstellungsbeispielen genannten Verbindungen die folgenden substituierten Acrylophenone der allgemeinen Formel (I) genannt:

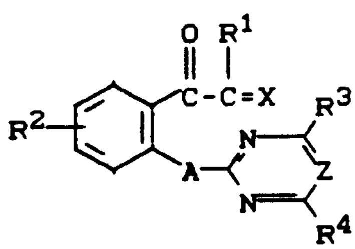

(I)

**Tabelle 1:**

| $R^1$ | $R^2$ | $R^3$ | $R^4$ | A | X | Z |
|---|---|---|---|---|---|---|
| H | H | $CH_3O$ | $CH_3O$ | O | $CH-N(C_2H_5)_2$ | CH |
| H | H | $CH_3O$ | $CH_3O$ | O | $CH-N[(CH_2)_2-CH_3]_2$ | CH |
| H | H | $CH_3O$ | $CH_3O$ | O | 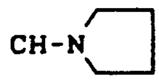 | CH |
| H | H | $CH_3O$ | $CH_3O$ | O | 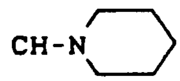 | CH |
| H | H | $CH_3O$ | $CH_3O$ | O | 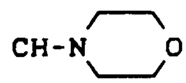 | CH |
| $CH_3$ | H | $CH_3O$ | $CH_3O$ | O | 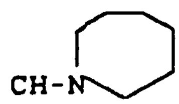 | CH |
| H | 4-Cl | $CH_3O$ | $CH_3O$ | O | 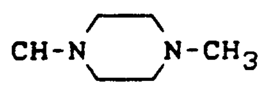 | CH |
| $C_2H_5$ | H | $CH_3O$ | $CH_3O$ | O | $CH-N(CH_3)_2$ | CH |
| H | H | $CH_3O$ | $CH_3$ | O | $CH-N(C_2H_5)_2$ | CH |
| H | H | $CH_3O$ | $CH_3$ | O | 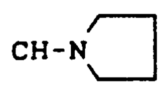 | CH |
| $CH_3$ | H | $CH_3O$ | $CH_3$ | O | $CH-N(CH_3)_2$ | CH |
| H | 3-$CH_3$ | $CH_3$ | $CH_3$ | O | 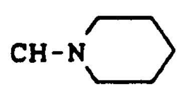 | CH |
| H | H | $CH_3$ | $CH_3$ | O | 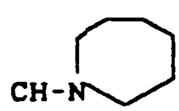 | CH |

Tabelle 1 - Fortsetzung

| $R^1$ | $R^2$ | $R^3$ | $R^4$ | A | X | Z |
|---|---|---|---|---|---|---|
| H | H | $CH_3$ | $CH_3$ | O | $CH-N(CH_3)_2$ | CH |
| H | H | $CH_3$ | $CH_3$ | O | $CH-N(C_2H_5)_2$ | CH |
| H | H | $CH_3$ | $CH_3$ | O | CH-N 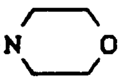 | CH |
| $CH_3$ | H | $CH_3$ | $CH_3$ | O | CH-N 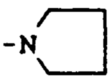 | CH |
| H | $4-C_2H_5$ | $CH_3$ | $CH_3$ | O | $CH-N(CH_3)_2$ | CH |
| H | H | $CH_3$ | $CH_3$ | O | $CH-N(CH_3)_2$ | $C-CH_3$ |
| H | H | $CH_3$ | $CH_3$ | O | $CH-N(CH_3)_2$ | $C-C_2H_5$ |
| H | H | $CF_3$ | $CH_3$ | O | $CH-N(CH_3)_2$ | CH |
| H | H | $CHF_2$ | $CHF_2$ | O | $CH-N(CH_3)_2$ | CH |
| H | H | $C_2H_5O$ | $C_2H_5O$ | O | $CH-N(CH_3)_2$ | CH |
| H | H | $C_2H_5O$ | $C_2H_5O$ | O | $CH-N(C_2H_5)_2$ | CH |
| H | H | $C_2H_5O$ | $CH_3$ | O | $CH-N(CH_3)_2$ | CH |
| H | 6-Cl | $CH_3O$ | $CH_3O$ | O | CH-N 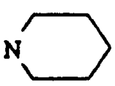 | CH |
| $C_2H_5$ | H | $CH_3O$ | $CH_3O$ | O | $CH-N(CH_3)_3$ | CH |
| H | H | $CH_3O$ | $CH_3O$ | O | 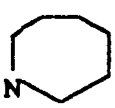 CH-N | $C-CH_3$ |
| H | H | $CH_3O$ | $CH_3O$ | S | $CH-N(CH_3)_2$ | CH |

<u>Tabelle 1</u> - Fortsetzung

| $R^1$ | $R^2$ | $R^3$ | $R^4$ | A | X | Z |
|---|---|---|---|---|---|---|
| H | H | $CH_3O$ | $CH_3O$ | S | CH-N 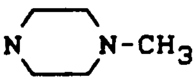 | CH |
| H | H | $CH_3O$ | $CH_3O$ | S | CH-N 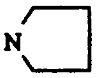 | CH |
| H | H | $CH_3$ | $CH_3$ | O | $CH-N(CH_3)_2$ | N |
| H | H | $CH_3$ | $CH_3$ | O | $CH-N(CH_3)_2$ | N |
| H | 3-F | $CH_3$ | $CH_3$ | O | CH-N 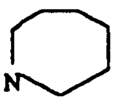 | N |
| H | 5-$CH_3$ | $CH_3$ | $CH_3$ | O | CH-N 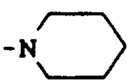 | N |
| H | H | $CH_3O$ | $CH_3$ | O | $CH-N(CH_3)_2$ | N |
| H | H | $CH_3O$ | $CH_3$ | O | CH-N 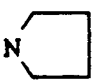 | N |
| $CH_3$ | H | $CH_3O$ | $CH_3$ | O | $CH-N(CH_3)_2$ | N |
| H | H | $CH_3O$ | $CH_3O$ | O | $CH-N(CH_3)_2$ | N |
| H | H | $C_2H_5O$ | $C_2H_5O$ | O | $CH-N(CH_3)_2$ | N |
| H | H | $C_2H_5$ | $C_2H_5$ | O | $CH-N(CH_3)_2$ | N |
| H | H | $CH_3S$ | $CH_3$ | O | $CH-N(CH_3)_2$ | N |
| H | H | $C_2H_5S$ | $CH_3$ | O | $CH-N(CH_3)_2$ | N |
| H | H | $CF_3O$ | $CH_3$ | O | $CH-N(CH_3)_2$ | N |
| H | H | $CF_3S$ | $CH_3$ | O | $CH-N(CH_3)_2$ | N |

Tabelle 1 - Fortsetzung

| $R^1$ | $R^2$ | $R^3$ | $R^4$ | A | X | Z |
|---|---|---|---|---|---|---|
| H | H | $F_2ClCS$ | $CH_3$ | O | $CH-N(CH_3)_2$ | N |
| H | H | $CH_3O$ | $CH_3O$ | O | 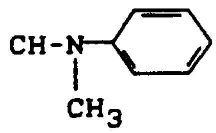 | CH |
| H | H | $CH_3O$ | $CH_3O$ | O | 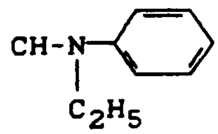 | CH |
| H | H | $CH_3O$ | $CH_3O$ | O | 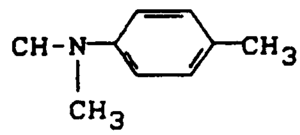 | CH |
| H | H | $CH_3O$ | $CH_3$ | O | 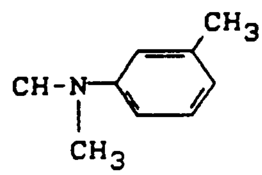 | CH |
| H | H | $CH_3O$ | $CH_3O$ | O | 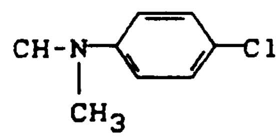 | CH |
| $CH_3$ | H | $CH_3O$ | $CH_3O$ | O | $P(C_6H_5)_3$ | CH |
| H | H | $CH_3O$ | $CH_3O$ | S | $P(C_6H_5)_3$ | CH |
| H | H | $CH_3O$ | $CH_3O$ | O | $P(C_6H_5)_3$ | $C-CH_3$ |
| H | H | $CH_3O$ | $CH_3O$ | O | 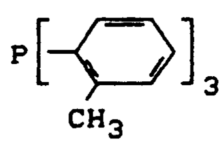 | CH |
| H | H | $CH_3$ | $CH_3$ | O | 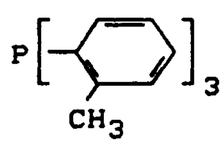 | N |

<u>Tabelle 1</u> - Fortsetzung

| $R^1$ | $R^2$ | $R^3$ | $R^4$ | A | X | Z |
|---|---|---|---|---|---|---|
| H | H | $CH_3O$ | $CH_3O$ | O | 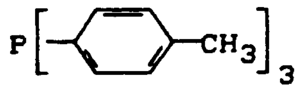 | CH |
| H | H | $CH_3O$ | $CH_3$ | O | 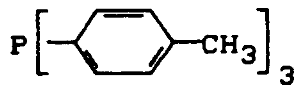 | CH |
| H | H | $CH_3O$ | $CH_3$ | O | $P(C_6H_5)_3$ | N |
| H | H | $CH_3O$ | $CH_3O$ | S | $P(C_6H_5)_3$ | N |

Verwendet man beispielsweise 3-(1-Hexahydroazepino)-1-(2-hydroxyphenyl)-prop-2-en-1-on und 4,6-Dimethoxy-2-methylsulfonylpyrimidin als Ausgangsstoffe, so läßt sich der Reaktionsablauf des erfindungsgemäßen Verfahrens (a) durch das folgende Formelschema darstellen:

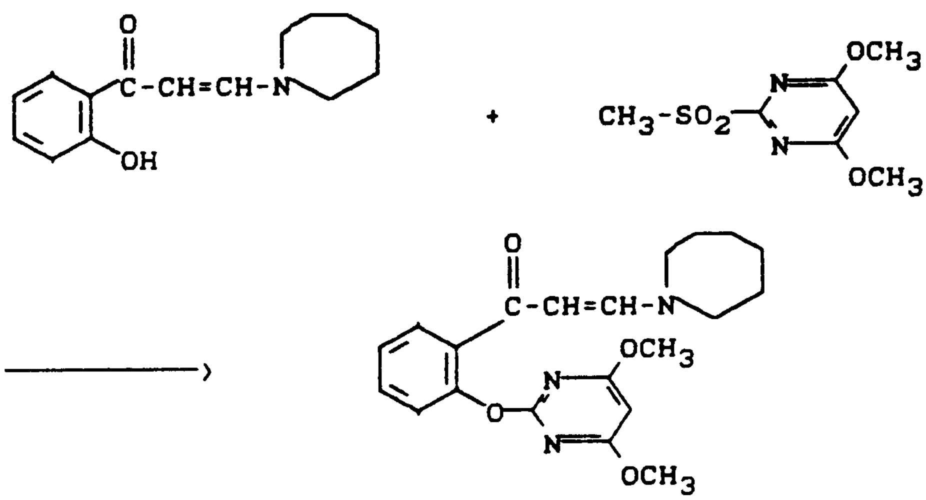

Verwendet man beispielsweise 2-[(2-Propionyl)-phenoxy)-4,6-dimethoxypyrimidin und N,N-Dimethylformamiddimethylacetal als Ausgangsstoffe, so läßt sich der Reaktionsablauf des erfindungsgemäßen Verfahrens (b) durch das folgende Formelschema darstellen:

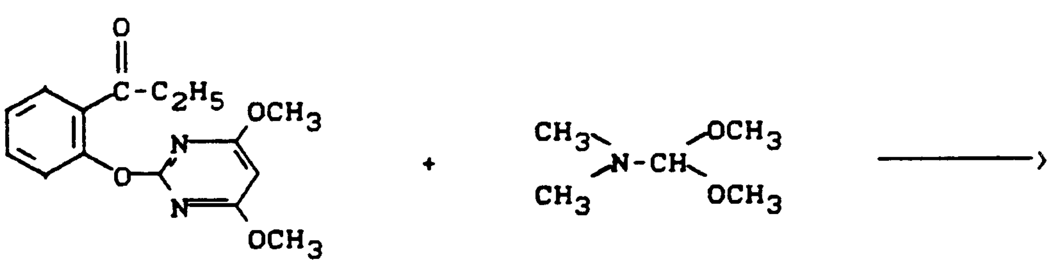

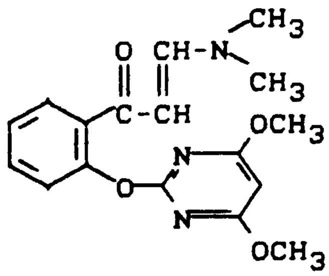

Die zur Durchführung des erfindungsgemäßen Verfahrens (a) als Ausgangsstoffe benötigten (Thio)-Phenolderivate sind durch die Formel (II) allgemein definiert. In dieser Formel (II) stehen $R^1$, $R^2$, A und X vorzugsweise für diejenigen Reste, die bereits im Zusammenhang mit der Beschreibung der erfindungsgemäßen Stoffe der Formel (I) als bevorzugt für diese Substituenten genannt wurden.

Die (Thio)Phenolderivate der Formel (II) sind bekannt (vgl. z.B. Rocz. Chem. 47, 841-852 [1973], J, Chem. Soc., Perkin Trans. 1, 1987, 2597-2604; EP 248420; Arch. Pharm, 318, 239-243, [1985]; DE 3228549; Synthesis 1982, 597-598; FR 2487357; Arch. Pharm. 314, 223-227 (1981]; Synthesis 1979, 901-903; Rocz. Chem. 40, 1683-1696 [1966)) oder erhältlich in Analogie zu bekannten Verfahren (vgl. auch die Herstellungsbeispiele).

Die zur Durchführung des erfindungsgemäßen Verfahrens (a) weiterhin als Ausgangsstoffe benötigten Heterocyclen sind durch die Formel (III) allgemein definiert. In dieser Formel (III) stehen $R^3$, $R^4$ und Z vorzugsweise für diejenigen Reste, die bereits im Zusammenhang mit der Beschreibung der erfindungsgemäßen Stoffe der Formel (I) als bevorzugt für diese Substituenten genannt wurden.

E steht vorzugsweise für Halogen oder Alkylsulfonyl, insbesondere für Chlor, Brom oder Methylsulfonyl.

Die Heterocyclen der Formel (III) sind ebenfalls bekannt (vgl. z.B. PCT Int. Appl. WO 8704321; EP 94260; DE 2656183; J, Chem, Soc. C, 1966, 2031-2038; JP 63023870) oder erhältlich in Analogie zu bekannten Verfahren.

Die zur Durchführung des erfindungsgemäßen Verfahrens (b) als Ausgangsstoffe benötigten Ketone sind durch die Formel (IV) allgemein definiert. In dieser Formel (IV) stehen $R^1$, $R^2$, $R^3$, $R^4$, A und Z vorzugsweise für diejenigen Reste, die bereits im Zusammenhang mit der Beschreibung der erfindungsgemäßen Stoffe der Formel (I) als bevorzugt für diese Substituenten genannt wurden.

Die Ketone der Formel (IV) sind teilweise bekannt (vgl. z.B. EP 336494) oder erhältlich in Analogie zu bekannten Verfahren, beispielsweise wenn man die allgemein bekannten Acyl(thio)-phenolderivate der Formel (VI),

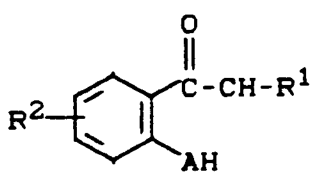

(VI)

in welcher

$R^1$, $R^2$ und A  die oben angegebene Bedeutung haben,

mit Heterocyclen der Formel (III),

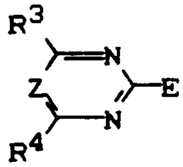

(III)

in welcher

E  für eine elektronenanziehende Abgangsgruppe steht und

$R^3$, $R^4$ und Z  die oben angegebene Bedeutung haben,

gegebenenfalls in Gegenwart eines Verdünnungsmittels wie beispielsweise Dimethylformamid und gegebenenfalls in Gegenwart eines Reaktionshilfsmittels wie beispielsweise Kaliumcarbonat bei Temperaturen zwischen 60° und 100° C umsetzt.

Die zur Durchführung des erfindungsgemäßen Verfahrens (b) weiterhin als Ausgangsstoffe benötigten Formamidacetale sind durch die Formel (V) allgemein definiert. In dieser Formel (V) stehen $R^5$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

$R^8$ steht vorzugsweise für geradkettiges oder verzweigtes Alkyl mit 1 bis 4 Kohlenstoffatomen, insbesondere für Methyl.

Die Formamidacetale der Formel (V) sind allgemein bekannte Verbindungen der organischen Chemie.

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether wie Diethylether, Dioxan, Tetrahydrofuran oder Ethylenglykoldimethyl- oder -diethylether, Ketone wie Aceton oder Butanon, Nitrile wie Acetonitril oder Propionitril, Amide wie Dimethylformamid, Dimethylacetamid, N-Methylformanilid, N-Methylpyrrolidon oder Hexamethylphosphorsäuretriamid, Ester wie Essigsäureethylester oder Sulfoxide wie Dimethylsulfoxid.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 20° C und 180° C, vorzugsweise bei Temperaturen zwischen 60° C und 100° C.

Zur Durchführung des erfindungsgemäßen Verfahrens (a) setzt man pro Mol an (Thio)Phenolderivat der Formel (II) im allgemeinen 1.0 bis 2.0 Mol, vorzugsweise 1.0 bis 1.2 Mol an Heterocyclus der Formel (III) und gegebenenfalls 1.0 bis 2.0 Mol, vorzugsweise 1.0 bis 1.2 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Die Reaktionstemperaturen können bei der Durchführung des erfindungsgemäßen Verfahrens (b) in einem größeren Bereich variiert werden. Im allgemeinen arbeitet man bei Temperaturen zwischen 20° C und 180° C, vorzugsweise bei Temperaturen zwischen 80° C und 140° C.

Zur Durchführung des erfindungsgemäßen Verfahrens (b) setzt man pro Mol an Keton der Formel (IV) im allgemeinen 1.0 bis 20.0 Mol, vorzugsweise 1.0 bis 5.0 Mol an Formamidacetal der Formel (V) ein.
Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von monokotylen und dikotylen Unkräutern in monokotylen und dikotylen Kulturen wie beispielsweise Weizen oder Sonnenblumen einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitro-benzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyl-iden]-cyclohexancarbonsäure (ALLOXYDIM); 4-Aminobenzolsulfonyl-methylcarbamat (ASULAM); 2-Chlor-4-ethylamino-6-isopropylamino-1,3,5-triazin (ATRAZIN);

2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiocarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxyl-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 2-Chlor-N-isopropylacetanilid (PROPACHLOR); Isopropyl-N-phenylcarbamat (PROPHAM); O-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäuremethylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden, Die Anwendung geschieht in üblicher Weise, z.B, durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab, Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

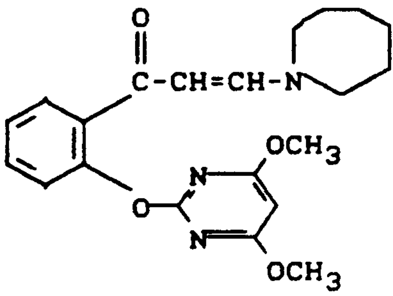

(Verfahren a)

5,6 g (0.02 Mol) 3-(1-Hexahydroazepino)-1-(2-hydroxyphenyl)-prop-2-en-1-on, 4.4 g (0.02 Mol) 4,6-Dimethoxy-2-methylsulfonylpyrimidin und 3,3 g (0.024 Mol) Kaliumcarbonat werden in 50 ml Acetonitril 16 Stunden auf Rückflußtemperatur erhitzt. Zur Aufarbeitung wird die erkaltete Reaktionsmischung im Vakuum eingeengt, der Rückstand in Methyl-t-butyl-ether aufgenommen, mit Wasser gewaschen, über Natriumsulfat getrocknet und im Hochvakuum von flüchtigen Anteilen befreit.

Man erhält 6.5 g (85% der Theorie) an 3-(1-Hexahydroazepino)-1-{2-[2-(4,6-dimethoxy)-pyrimidinyloxy]-phenyl}-prop-2-en-1-on als Öl.

$^1$H-NMR ($CDCl_3$/Tetramethylsilan): $\delta$ = 3.8 (s, 6H) ppm.

Herstellung der Ausgangsverbindung

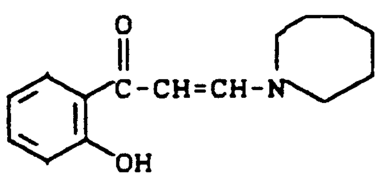

20.4 g (0.15 Mol) 2-Hydroxyacetophenon und 31.3 g (0.18 Mol) N-Formylhexamethyleniminmdimethylacetal werden 16 Stunden auf Rückflußtemperatur erhitzt, wobei freiwerdendes Methanol kontinuierlich abdestilliert wird. Zur Aufarbeitung wird die erkaltete Reaktionsmischung im Hochvakuum von flüchtigen Bestandteilen befreit.

Man erhält 38.3 g (100% der Theorie) an 3-(1-Hexahydroazepino)-1-(2-hydroxyphenyl)-prop-2-en-1-on als Öl.

$^1$H-NMR ($CDCl_3$/Tetramethylsilan): $\delta$ = 5.8 (d, 1H) ppm.

Beispiel 2:

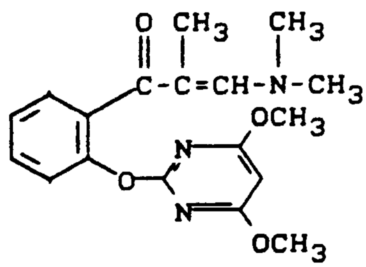

(Verfahren b)

1 g (0.0035 Mol) 2-(2-Propionylphenoxy)-4,6-dimethoxypyrimidin und 0.5 g (0.0042 Mol) N,N-Dimethylformamid-dimethylacetal werden 16 Stunden auf Rückflußtemperatur erhitzt, abgekühlt und im Hochvakuum von flüchtigen Bestandteilen befreit.

Man erhält 1.1 g (92% der Theorie) an 3-Dimethylamino-2-methyl-1-{2-[2-(4,6-dimethoxy)-pyrimidinyloxy]-phenyl}-prop-2-en-1-on als Öl.

$^1$H-NMR ($CDCl_3$/Tetramethylsilan) $\delta$ = 3.8 (s, 6H) ppm.

Herstellung der Ausgangsverbindung:

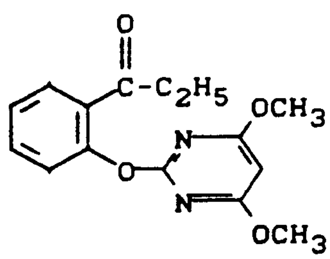

3.8 g (0.025 Mol) o-Hydroxypropiophenon, 5.5 g (0.0025 Mol) 4,6-Dimethoxy-2-methylsulfonylpyrimidin und 4.1 g Kaliumcarbonat werden in 50 ml Acetonitril 16 Stunden auf Rückflußtemperatur erhitzt. Zur Aufarbeitung wird auf Raumtemperatur abgekühlt, im Vakuum eingeengt, der Rückstand in Dichlormethan aufgenommen, mit Wasser gewaschen, über Natriumsulfat getrocknet, eingeengt und an Kieselgel (Laufmittel: Dichlormethan) chromatographiert.

Man erhält 3.2 g (44% der Theorie) an 2-(2-Propionylphenoxy)-4,6-dimethoxypyrimidin vom Schmelzpunkt 80 ° C.

Beispiel 3:

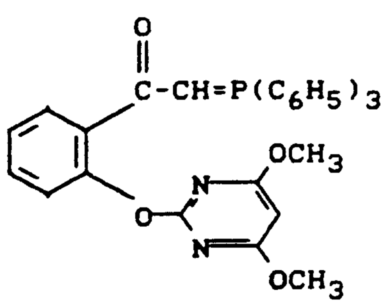

(Verfahren a)

4.8 g (0.012 Mol) (2-Hydroxybenzoyl)methylen-triphenylphosphoran, 2.6 g (0.012 Mol) 4,6-Dimethoxy-2-methylsulfonyl-pyrimidin und 2.0 g (0.0144 Mol) Kaliumcarbonat werden in 50 ml trockenem Acetonitril 48 Stunden auf Rückflußtemperatur erhitzt, anschließend auf Raumtemperatur abgekühlt, im Vakuum eingeengt, der Rückstand in Dichlormethan aufgenommen, mit Wasser gewaschen, über Natriumsulfat getrocknet und eingeengt. Der Rückstand wird mit Ether verrührt, kristalliner Niederschlag abgesaugt, das Filtrat eingeengt und über Kieselgel (Laufmittel: Hexan/Aceton 7:3) chromatographiert.

Man erhält als 2. Fraktion 0.8 g (12% der Theorie) an {2-[2-(4,6-Dimethoxy)-pyrimidinyloxy]-benzoyl}-methylentriphenylphosphoran vom Schmelzpunkt 118°-120°C.

In entsprechender Weise und gemäß den allgemeinen Angaben zur Herstellung erhält man die folgenden substituierten Acrylophenone der allgemeinen Formel (I):

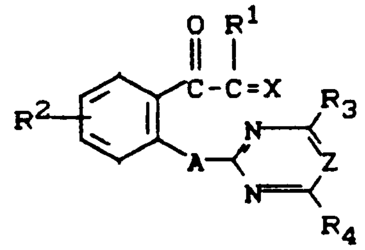

(I)

| Bsp.-Nr. | $R^1$ | $R^2$ | $R^3$ | $R^4$ | A | X | Z | Physikalische Eigenschaften |
|---|---|---|---|---|---|---|---|---|
| 4 | H | H | $OCH_3$ | $OCH_3$ | O | $CH-N(CH_3)_2$ | CH | $^1H$-NMR*): 5.6 (d, 1H) |
| 5 | H | 5-Cl | $OCH_3$ | $OCH_3$ | O | $CH-N(CH_3)_2$ | CH | Fp: 143° C |
| 6 | H | $5-CH_3$ | $OCH_3$ | $OCH_3$ | O | $CH-N(CH_3)_2$ | CH | Fp: 140° C |
| 7 | H | H | $OCH_3$ | $CH_3$ | O | $CH-N(CH_3)_2$ | CH | Fp: 91° C |
| 8 | H | H | $CH_3$ | $CH_3$ | O | $CH-N(CH_3)_2$ | CH | Fp.: 140°C |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele:

In den folgenden Anwendungsbeispielen wurde die nachstehend aufgeführte Verbindung als Vergleichs-

substanz eingesetzt:

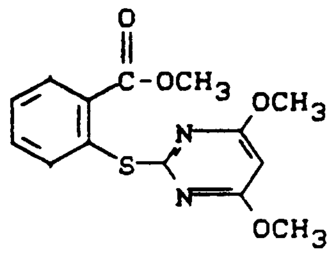

(A)

2-[2-(4,6-Dimethoxy)-pyrimidinylthio]-benzoesäuremethylester (bekannt aus EP 223406/Bsp. 11).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß folgender Herstellungsbeispiele: 1 und 4.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 1, 2, 3 und 4.

## Patentansprüche

**1.** Acrylophenone der allgemeinen Formel

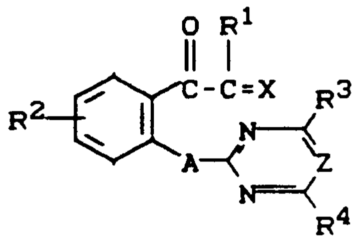

(I)

in welcher

| | |
|---|---|
| $R^1$ | für Wasserstoff oder Alkyl steht, |
| $R^2$ | für Wasserstoff, Halogen oder Alkyl steht, |
| $R^3$ und $R^4$ | unabhängig voneinander jeweils für Wasserstoff, Alkyl, Alkoxy, Alkylthio, Halogenalkyl, Halogenalkoxy oder Halogenalkylthio stehen, |
| A | für Sauerstoff oder Schwefel steht, |
| X | für einen Rest |

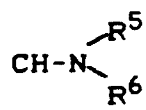

oder für einen Rest $P(Ar)_3$ steht und

| | |
|---|---|
| Z | für Stickstoff oder für einen Rest $C\text{-}R^7$ steht, |

wobei entweder

| | |
|---|---|
| $R^5$ | für Wasserstoff oder Alkyl steht und |
| $R^6$ | für Alkyl oder für gegebenenfalls substituiertes Phenyl steht oder |
| $R^5$ und $R^6$ | gemeinsam mit dem Stickstoffatom, an welches sie gebunden sind, für einen gegebenenfalls substituierten gesättigten Heterocyclus stehen, der gegebenenfalls weitere Heteroatome enthalten kann, |
| $R^7$ | für Wasserstoff oder Alkyl steht und |
| Ar | für gegebenenfalls substituiertes Phenyl steht, |

**2.** Acrylophenone der Formel (I) gemäß Anspruch 1,

in welcher

| | |
|---|---|
| $R^1$ | für Wasserstoff oder für geradkettiges oder verzweigtes Alkyl mit 1 bis 4 Kohlenstoffatomen steht, |
| $R^2$ | für Wasserstoff, Fluor, Chlor, Brom, Jod oder für geradkettiges oder verzweigtes Alkyl mit 1 bis 4 Kohlenstoffatomen steht, |
| $R^3$ und $R^4$ | unabhängig voneinander jeweils für Wasserstoff, für jeweils geradkettiges oder verzweigtes Alkyl, Alkoxy oder Alkylthio mit jeweils 1 bis 4 Kohlenstoffatomen oder für jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen stehen, |
| A | für Sauerstoff oder Schwefel steht, |
| X | für einen Rest |

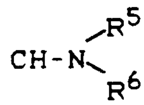

oder für einen Rest $P(Ar)_3$ steht und

| | |
|---|---|
| Z | für Stickstoff oder für einen Rest $C\text{-}R^7$ steht, |

wobei entweder

$R^5$ für Wasserstoff oder für geradkettiges oder verzweigtes Alkyl mit 1 bis 4 Kohlenstoffatomen steht und

$R^6$ für geradkettiges oder verzweigtes Alkyl mit 1 bis 4 Kohlenstoffatomen oder für gegebenenfalls einfach oder mehrfach gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, oder

$R^5$ und $R^6$ gemeinsam mit dem Stickstoffatom, an welches sie gebunden sind, für einen gegebenenfalls einfach oder mehrfach, gleich oder verschieden durch geradkettiges oder verzweigtes Alkyl mit 1 bis 4 Kohlenstoffatomen substituierten gesättigten fünf- bis siebengliedrigen Heterocyclus stehen, der gegebenenfalls ein weiteres Heteroatom - insbesondere Stickstoff, Sauerstoff oder Schwefel - enthalten kann,

$R^7$ für Wasserstoff oder für geradkettiges oder verzweigtes Alkyl mit 1 bis 4 Kohlenstoffatomen steht und

Ar für gegebenenfalls einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen.

**3.** Acrylophenone der Formel (I) gemäß Anspruch 1,

in welcher

$R^1$ für Wasserstoff, Methyl oder Ethyl steht,

$R^2$ für Wasserstoff, Fluor, Chlor, Brom, Methyl oder Ethyl steht,

$R^3$ und $R^4$ unabhängig voneinander jeweils für Wasserstoff, Methyl, Ethyl, n- oder i-Propyl, Methoxy, Ethoxy, Methylthio, Ethylthio, Trifluormethyl, Difluormethyl, Trichlormethyl, Trifluormethoxy, Difluormethoxy, Trifluormethylthio, Trichlormethylthio, Chlordifluormethylthio oder Dichlorfluormethylthio stehen,

A für Sauerstoff oder Schwefel steht,

X für einen Rest

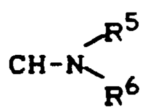

oder für einen Rest $P(Ar)_3$ steht und

Z für Stickstoff oder für einen Rest $C-R^7$ steht,

wobei entweder

$R^5$ für Methyl, Ethyl, sowie n- oder i-Propyl und

$R^6$ für Methyl, Ethyl, n- oder i-Propyl oder für gegebenenfalls einfach oder zweifach gleich oder verschieden substituiertes Phenyl steht, wobei als Substituenten infrage kommen: Fluor, Chlor, Brom, Methyl, Ethyl, Methoxy, Ethoxy oder Trifluormethyl oder

$R^5$ und $R^6$ gemeinsam mit dem Stickstoffatom, an welches sie gebunden sind, für einen gegebenenfalls ein- bis dreifach, gleich oder verschieden durch Methyl, Ethyl sowie n- oder i-Propyl substituierten Heterocyclus der Formel

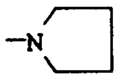 ; 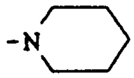 ; 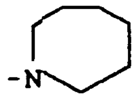 ; 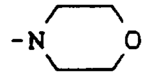 ;

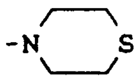 oder 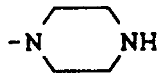

stehen,

$R^7$ für Wasserstoff, Methyl oder Ethyl steht und

Ar für gegebenenfalls einfach oder zweifach, gleich oder verschieden substituiertes Phenyl steht, wobei als Substituenten infrage kommen: Fluor, Chlor, Brom, Methyl, Ethyl, Methoxy, Ethoxy oder Trifluormethyl.

**4.** Verfahren zur Herstellung von Acrylophenonen der allgemeinen Formel (I)

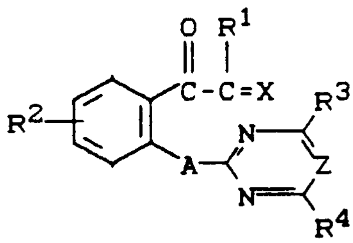

(I)

in welcher

$R^1$ für Wasserstoff oder Alkyl steht,

$R^2$ für Wasserstoff, Halogen oder Alkyl steht,

$R^3$ und $R^4$ unabhängig voneinander jeweils für Wasserstoff, Alkyl, Alkoxy, Alkylthio, Halogenalkyl, Halogenalkoxy oder Halogenalkylthio stehen,

A für Sauerstoff oder Schwefel steht,

X für einen Rest

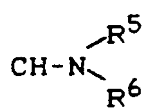

oder für einen Rest $P(Ar)_3$

steht und

Z für Stickstoff oder für einen Rest C-$R^7$ steht,

wobei entweder

$R^5$ für Wasserstoff oder Alkyl steht und

$R^6$ für Alkyl oder für gegebenenfalls substituiertes Phenyl steht oder

$R^5$ und $R^6$ gemeinsam mit dem Stickstoffatom, an welches sie gebunden sind, für einen gegebenenfalls substituierten gesättigten Heterocyclus stehen, der gegebenenfalls weitere Heteroatome enthalten kann,

$R^7$ für Wasserstoff oder Alkyl steht und

Ar für gegebenenfalls substituiertes Phenyl steht,

dadurch gekennzeichnet, daß man

(a) (Thio)Phenolderivate der Formel (II),

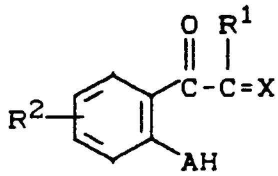

(II)

in welcher

$R^1$, $R^2$, A und X die oben angegebene Bedeutung haben,

mit Heterocyclen der Formel (III),

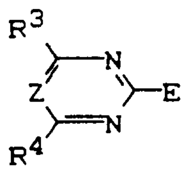

(III)

in welcher

E für eine elektronenanziehende Abgangsgruppe steht und

$R^3$, $R^4$ und Z die oben angegebene Bedeutung haben,

gegebenenfalls in Gegenwart eines Verdünnungsmittels und gegebenenfalls in Gegenwart eines Reaktionshilfsmittels umsetzt oder

(b) Ketone der Formel (IV),

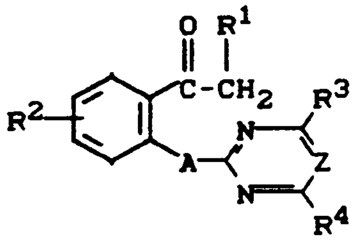

(IV)

in welcher

$R^1$, $R^2$, $R^3$, $R^4$, A und Z die oben angegebene Bedeutung haben,

mit Formamidacetalen der Formel (V),

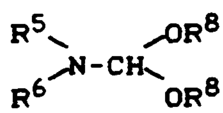

(V)

in welcher

$R^8$ für Alkyl steht und

$R^5$ und $R^6$ die oben angegebene Bedeutung haben,

gegebenenfalls in Gegenwart eines Verdünnungsmittels umsetzt.

**5.** Herbizide Mittel, gekenzeichnet durch einen Gehalt an mindestens einem Acrylophenon der Formel (I) gemäß den Ansprüchen 1 bis 4.

**6.** Verfahren zur Bekämpfung von Unkräutern, dadurch gekennzeichnet, daß man Acrylophenone der Formel (I) gemäß den Ansprüchen 1 bis 4 auf die Unkräuter und/oder ihren Lebensraum einwirken läßt.

7. Verwendung von Acrylophenonen der Formel (I) gemäß den Ansprüchen 1 bis 4 zur Bekämpfung von Unkräutern.

8. Verfahren zur Herstellung von herbiziden Mitteln, dadurch gekennzeichnet, daß man Acrylophenone der Formel (I) gemäß den Ansprüchen 1 bis 4 mit Streckmitteln und/oder oberflächenaktiven Substanzen vermischt.